# EUROPEAN PATENT SPECIFICATION

(11) **EP 3 962 561 B1**
(45) Date of publication and mention of the grant of the patent: **11.02.2026**
(21) Application number: 20722282.9
(22) Date of filing: 24.04.2020
(51) Int. Cl.: B26D 1/06, B26D 5/08, B26D 7/06, A61M 25/00, B26D 7/01, A61M 25/01

(54) **FLEXIBLE ELONGATED TUBE FOR A CATHETER SYSTEM**
FLEXIBLES LANGGESTRECKTES ROHR FÜR EIN KATHETERSYSTEM
TUBE FLEXIBLE ALLONGÉ POUR SYSTÈME DE CATHÉTER

(30) Priority: 30.04.2019 US 201962840415 P; 22.05.2019 EP 19175878
(43) Date of publication of application: 09.03.2022
(73) Proprietor: Biotronik AG, 8180 Bülach (CH)
(72) Inventor: BURKE, Jeff Alan, Fallbrook, CA 92028 (US); D'AQUANNI, Peter, Murrieta, CA 92562 (US); DENISON, Andy Edward, Temecula, CA 92591 (US); HAYDEN, Christopher Scott, Winchester, CA 92596 (US); FÖRSTER, Markus, 5105 Auenstein (CH); WALTHER, Michael, 8400 Winterthur (CH); FREY, Lukas, 4900 Langenthal (CH)
(74) Representative: WSL Patentanwälte Partnerschaft mbB
(86) International application number: PCT/EP2020/061441
(87) International publication number: WO 2020/221664

(56) References cited:
- US-A- 3 149 186
- US-A1- 2006 259 124
- US-A1- 2013 096 553
- US-A1- 2014 053 940
- US-A1- 2017 173 303

## Description

The present invention relates to a flexible elongated tube for a catheter system according to the preamble of claim 1, to a catheter arrangement comprising such a flexible elongated tube according to the preamble of claim 5, to a method for manufacturing such a flexible elongated tube according to the preamble of claim 8, and to an indentation tool for manufacturing such a flexible elongated tube according to the preamble of claim 13.

Flexible elongated tubes are often used in catheter systems as catheter shanks or catheter shafts. To achieve a deflection of such flexible catheter shaft, a pull wire is typically used that is arranged inside the catheter shaft. Such a pull wire is tightly connected at the distal end of the catheter shaft and can be operated by a handle or a comparable operating means at a proximal end of the catheter shaft. By applying tension to the pull wire, deflection of the catheter shaft results.

Prior art discloses different possibilities of arranging a pull wire within an elongated flexible tube acting as a catheter shaft. Thereby, these different concepts aim at constraining the pull wire within the catheter shaft, but allowing sufficient flexibility of the pull wire so that it can be used for deflecting the catheter shaft.

WO 2017/142778 A1 describes a deflectable catheter shaft comprising a plurality of tabs forming pull wire tunnels. Thereby, one pull wire is guided regularly between a plurality of tabs and an interior wall of a flexible elongated tube forming a catheter shaft.

A similar solution is known from US 9,795,765 B2. However, these solutions are somewhat problematic since a comparably high friction is applied onto the pull wire when it is moved within the formed pull wire tunnel since it abuts sharp edges both of the tabs and of the wall of the catheter shaft.

US 8,721,826 B2 proposes to arrange the pull wire within the inner lumen of a catheter shaft. However, in such a case, wiggling of the pull wire and of the catheter shaft can occur. This US patent also proposes to apply a different shape for the catheter shaft in order to reduce the negative wiggling effects. Such a solution, however, is connected to significant higher efforts for producing the catheter shaft.

EP 0 630 657 B1 discloses a catheter shaft in which the pull wire is guided through an additional guiding tube. While the friction acting on the pull wire is reduced by this solution, the manufacturing costs and efforts are significantly increased.

US 2006/0259124 A1 describes a stent delivery device including a distal-side tube having a guide wire lumen; a proximal-side tube whose distal portion is fixed to a proximal portion of the distal-side tube; a cylindrical member which encloses a distal side of the distal-side tube and is slidable toward a proximal end of the distal-side tube; a stent accommodated in the cylindrical member; and a pulling member for moving the cylindrical member toward a proximal side of the stent delivery device. The distal-side tube has a proximal-side opening which is open at the proximal side of the distal-side tube; and a stent-locking portion for preventing the stent from moving to the proximal side of the stent delivery device. The outer diameter of the proximal-side tube is set smaller than that of a portion, having a maximum diameter, which is disposed in a region of the stent delivery device which is distal from the proximal-side tube.

US 2013/0096553 A1 describes medical devices and methods for making and using medical devices. An example medical device may be a deflectable medical device that includes a catheter shaft having a distal end. An ablation electrode may be disposed at the distal end. A deflection mechanism may be coupled to the catheter shaft. The deflection mechanism may include a deflection body and a pull wire coupled to the deflection body. The deflection body may have a longitudinally-extending furrow formed therein. A flex member may be disposed adjacent to the deflection mechanism.

US 2014/0053940 A1 describes a bending tube including: multiple first slots for bending provided at set intervals, respectively, along a direction of a direction of a longitudinal axis O of a cylindrical bending tube body, the multiple first slots for bending extending in a circumferential direction of the bending tube body; slots for forming wire guide (a first slot for bending) paired and provided on arrangement of the multiple first slots for bending, the slots for forming wire guide extending in the circumferential direction of the bending tube body; and a wire guide formed by deforming a circumferential part of the bending tube body between the paired slots for forming wire guide, in an inner diameter direction; a width of a slot for bending adjacent to the wire guide is set relatively narrower than a width of other slots for bending.

US 3,149,186 A describes a method of making a catheter for peritoneal dialysis comprising: cutting a length of extruded, thermoplastic tubing, said tubing having the desired physical properties and including a bore defined by a generally cylindrical, semirigid, self-supporting wall; coating a pointed pin with silicone; heating said pin to a temperature above the softening point but below the melting point of the tubing; pressing the point of the heated pin against the outer surface of the tubing wall near one end of the tubing to form a permanent indentation; further pressing the point of the pin against the side wall forcing the pin to pierce said wall and enter the bore of the tubing; stopping the movement of said pin while the point is in the tube bore and before the point contacts the opposite wall of the tubing; retracting the pin from the tubing wall, the silicone serving to release the pin without causing projecting discontinuities on the outer surface of the tubing; forming a curved section along the perforated end portion of the tubing; and forming a smooth rounded tip on the end of said portion.

US2017/173303 A1 provides an example of a steerable medical device.

It is an object of the present invention to provide an elongated flexible tube that can be used as catheter shaft and that offers a guidance for a pull wire that can be easily manufactured and that puts less friction forces onto a pull wire when the pull wire is moved inside the catheter shaft.

This object is achieved with a flexible elongated tube for a catheter system having the features according to claim 1. Such a tube comprises a tubing wall, a plurality of first indentations of the tubing wall and a plurality of second indentations of the tubing wall. The first indentations are spaced apart from each other along a longitudinal axis of the tube. Likewise, the second indentations are spaced apart from each other along the longitudinal axis of the tube.

One first indentation and one second indentation form in each case an axially aligned pair of indentations. Whenever the term "axially aligned" is used or if otherwise reference is made to an axis, the longitudinal axis of the tube is referred to, if not explicitly stated otherwise. A non-impressed section of the tubing is present between the axially aligned pair of indentations. Expressed in other words, if looking at a cross-section of the tube, there is a first indentation and a second indentation as well as a non-impressed section between the first indentation and the second indentation.

According to the presently claimed invention, a first inner surface of the first indentation and a second inner surface of the second indentation of the axially aligned pair of indentations form together with an inner surface of the non-impressed section of the tubing wall a pull wire space. This pull wire space is arranged and dimensioned such to receive and guide a pull wire within the flexible elongated tube. Thus, the dimensions of the pull wire space are adapted to restrict undesired movements of the pull wire and thus to avoid wiggling or snaking of the pull wire in the tube (either when activated or non-activated). On the other hand, the pull wire space is sufficiently big to allow a smooth movement of the pull wire within the pull wire space without putting significant friction forces onto the pull wire.

In contrast to prior art solutions, the pull wire is not radially clamped between a tab that is pushed into the inside of the catheter shaft and an inner wall of the catheter shaft. Rather, it is fully placed in an interior of the flexible elongated tube, wherein its lateral movements (i.e., movements of the pull wire along a circumference of the tube) are restricted by a plurality of axially aligned pairs of first indentations and second indentations.

The shape of the first indentations and the second indentations as well as a distance between the first indentation and the second indentation of one pair of axially aligned indentations determines the dimensions of the pull wire space. It can be easily adapted to different sizes of pull wires. Furthermore, the axial distance between individual first indentations and individual second indentations and thus the distance between different axially aligned pairs of a first indentation and a second indentation determines the guiding stiffness of the pull wire in the axial direction, i.e., along the direction of the longitudinal axis of the tube. The bigger the distance between individual axially aligned pairs of indentations the more flexible is a pull wire guided within the tube.

The tube can comprise or (essentially) consist of a metallic material. Nitinol and stainless steel are particularly appropriate metallic materials. Moreover, the tube can comprise a polymeric material. For instance, the tube may comprise a Polytetrafluoroethylene (PTFE) liner positioned inside the tube. Polytetrafluoroethylene (PTFE) is a particularly appropriate polymeric material.

In an embodiment, a coating of a friction-reducing material is applied onto an inner surface of the tube and/or the first indentations and/or the second indentations. Such a friction-reducing layer can, e.g., be applied in form of a coating or in form of a liner. Typical friction-reducing materials like polymers generally known *per se* to a person skilled in the art can well be used for such purposes. Polyether block amides and other thermoplastic elastomers, polyamides, fluorinated hydrocarbons such as PTFE and siloxanes like silicones are particularly appropriate friction-reducing materials.

In an embodiment, at least some, in particular all, first indentations of the plurality of first indentations and/or at least some, in particular all, second indentations of the plurality of second indentations are structurally identical, i.e., constructed in the same way. This reduces manufacturing efforts and allows for a symmetric appearance of the pull wire space.

In an embodiment, at least some, in particular all, first indentations of the plurality of indentations and/or at least some, in particular all, second indentations of the plurality of second indentations are separated from axially adjacent non-impressed sections of the flexible elongated tube by a cut. Such a cut facilitates a formation of the first indentations and the second indentations since the tubing wall sections to be indented or impressed can then be easily moved relatively to other tubing wall sections that shall remain non-impressed. Such a cut is typically not present between the first indentation and/or the second indentation of the pair of axially aligned indentations and the remaining non-impressed section of the tubing wall circumferentially arranged between these indentations. Thus, a cut is typically formed along a circumference of the tube, but not perpendicular to the circumference, i.e., not along a longitudinal axis of the tube.

In an embodiment, a gap is formed between at least some, in particular all, first indentations of the plurality of first indentations and axially adjacent non-impressed sections of the flexible elongated tube and/or between at least some, in particular all, second indentations of the plurality of second indentations and axially adjacent non-impressed sections of the flexible elongated tube. Thereby, the gap provides a fluid communication between an interior of the flexible elongated tube and an exterior thereof. Such a gap is typically formed when a wall section of the tube is impressed to form a first indentation or a second indentation. Depending on the amount of impression and on the wall thickness of the tubing wall, such a gap may be formed. It does not impair the functionality of the tube when, e.g., used as catheter shaft. However, it should be noted that such a gap is not intended to guide a pull wire through it. Rather, the gap is merely a result of using tubing wall for forming the first indentations and/or the second indentations.

In an aspect, the present invention relates to a catheter arrangement comprising a flexible elongated tube according to the preceding explanations as well as a pull wire being arranged inside this tube. Thereby, the flexible elongated tube comprises a tubing wall and a plurality of first indentations of the tubing wall as well as a plurality of second indentations of the tubing wall. The first indentations are spaced apart from each other along the longitudinal axis of the tube. Likewise, the second indentations are spaced apart from each other along the longitudinal axis of the tube.

As already explained above, in each case, one first indentation and one second indentation form an axially aligned pair of indentations. Thereby, "axially" relates to the longitudinal axis of the tube. A non-impressed section of the tubing remains present between the first indentation and the second indentation of the axially aligned pair of indentations.

According to the invention, a first inner surface of the first indentation and a second inner surface of the second indentation of the axially aligned pair of indentations as well as an inner surface of the non-impressed section of the tubing wall between the first indentation and the second indentation of the axially aligned pair of indentations form a pull wire space. The pull wire is received and guided in this pull wire space within the flexible elongated tube. Thus, the pull wire space forms a receptacle for the pull wire that, on the one hand, restricts undesired movements of the pull wire but, on the other hand, allows a friction-less easy movement of the pull wire in the pull direction to achieve a deflection of the flexible elongated tube. This enables an application of the flexible elongated tube as a catheter shaft of the catheter arrangement.

In an embodiment, an inner tube is additionally received in an interior of the flexible elongated tube. This inner tube can serve for guiding specific catheter elements, such as a guide wire or an implant, inside the flexible elongated tube.

In an embodiment, an outer surface of the inner tube forms an additional wall of the pull wire space and thus additionally limits the maximum possible movements of the pull wire received within the pull wire space. In a typical arrangement, the pull wire space is limited on a first side by the first indentation of the axially aligned pair of indentations, on a second side (in particular opposite the first side) by the second indentation of the pair of axially aligned indentations, on a third side (typically essentially perpendicular to the first side and the second side) by an inner wall of the non-impressed section of the tubing wall and on a fourth side (typically opposite the third side) by an outer surface of the inner tube. In such an arrangement, an undesired snaking of the pull wire is particularly appropriate suppressed. Nonetheless, the pull wire can be pulled in order to deflect the elongated flexible tube to a desired amount or angle.

**In** an aspect, the present invention relates to a method for manufacturing a flexible elongated tube according to the preceding explanations. This method comprises the steps explained in the following:
First, a flexible elongated tube is placed into a fixing device. This fixing device serves to fix and align the flexible elongated tube at least section-wise in an oblong aligning direction. This aligning direction corresponds to the longitudinal axis of the tube.

Then, a pusher is placed into a pusher guidance of the fixing device.

Subsequently, the pusher is advanced towards the flexible elongated tube in a guiding direction within the pusher guidance. This guidance direction is essentially perpendicular to the aligning direction. Thus, an advancement of the pusher along the guiding direction leads to an advancement of the pusher essentially perpendicular to the longitudinal axis of the tube.

The advancement of the pusher results in a contact of the gripper, which is arranged at an end face of the pusher, and the flexible elongated tube.

The pusher is further advanced in the guidance direction until it contacts the stop of the pusher guidance. This stop automatically limits the maximum approach of the pusher to the tube. **In** this defined position between the tube and the pusher, the gripper of the pusher automatically exerts a defined pressure onto the tube. This pressure leads to formation of the first indentation and the second indentation that are axially aligned with respect to the longitudinal axis of the flexible elongated tube. Thus, the gripper produces one first indentation and one second indentation (that are axially aligned) automatically at the same time. The depth of the indentations is preset by the dimensions of the pusher, the gripper and the maximum possible movement of the pusher in the guiding direction within the pusher guidance. The pusher is then retracted away from the flexible elongated tube. It can be fully removed from the pusher guidance. **In** an embodiment, however, it is only retracted from the tube to such an extent that a movement of the tube within the fixing device is possible.

Afterwards, the tube is advanced in the fixing device so that another portion of the tube is placed at that section of the fixing device in which the pusher guidance is present.

Then, the step of advancing the pusher towards the tube, contacting the tube with the gripper, advancing the pusher until it contacts the stop, retracting the pusher and advancing the tube within the fixing device are iteratively repeated so as to produce a plurality of first indentations and a plurality of second indentations in the tubing wall of the tube. Thereby, always one first indentation and one second indentation are axially aligned and form a pair of axially aligned indentations.

In an embodiment, the tube is advanced within the fixing device by an amount that can be predefined. The fixing device can be equipped with a means for allowing an easy advancement of the tube by the amount that can be predefined or preset.

In an embodiment, the pre-definable amount remains constant for a plurality of subsequent advancing steps. In doing so, equally distant axially aligned pairs of a first indentation and a second indentation can be particularly easy formed in the tube.

In an embodiment, the pusher is at least partially advanced with a clamp. With such a clamp, the necessary pressure to form the indentations into the tubing wall can be particularly easy applied.

In an embodiment, the first indentation and the second indentation of the axially aligned pair of indentations are formed in an area of the flexible elongated tube between at least two axially spaced cuts around a section of the circumference of the tube. As explained above, such a formation of indentations between two axially spaced cuts allows for a particularly easy impression of the tubing wall sections that are intended to form the indentations. By providing such cuts, an undesired and uncontrollable tearing of the tubing wall can be avoided. The cuts can be provided, e.g., by laser cutting. Laser cutting is a particular appropriate cutting method if a metallic material is used for the tubing wall. Such laser cuts can also be used to allow a (higher) flexibility of the tube so as to allow bending or deflection of the tube.

**In** an aspect, the present invention relates to an indentation tool that can be used for manufacturing a flexible elongated tube according to the preceding explanations. This indentation tool can particularly be used for carrying out the manufacturing method explained in the previous sections.

The indentation tool comprises a fixing device for fixing and aligning the flexible elongated tube at least section-wise in an oblong aligning direction. This aligning direction typically corresponds to a longitudinal axis of the flexible elongated tube. The fixing device further comprises a pusher guidance for guiding the pusher in a guidance direction. Thereby, the guidance direction is essentially perpendicular to the aligning direction.

The indentation tool further comprises a pusher. This pusher can be moved in the pusher guidance along the guidance direction. Thereby, the pusher comprises a gripper that is arranged on an end face of the pusher. The gripper is arranged and designed for making indentations into a flexible elongated tube that is arranged in the fixing device.

The pusher guidance comprises a stop for limiting the maximum possible movement of the pusher along the guiding direction in the pusher guidance towards the fixing device. Expressed in other words, the stop for limiting the maximum possible movement of the pusher defines a minimum distance between the pusher or the gripper of the pusher, respectively, and a virtual axis extending along the aligning direction centrally in an area of the fixing device in which a flexible elongated tube is to be placed. This virtual axis corresponds to a longitudinal axis of a flexible elongated tube that is to be placed into the fixing device. The dimensions of the pusher, the gripper and the stop of the pusher guidance can be adapted to different tubes to be placed into the fixing device so as to be able to manufacture differently designed indentations into differently dimensioned tubes.

The indentation tool enables the impression of 2 axially aligned indentations at the same time. By relative movements between its tube to be placed into the fixing device of the indentation tool and the indentation tool itself, a plurality of axially spaced pairs of indentations can be produced in a particularly easy and fast way.

In an embodiment, the fixing device comprises an inspection window. This inspection window allows for a visual control of the contact zone in which the gripper can contact the flexible elongated tube arranged in the fixing device during intended operation of the indentation tool. Then, the step of impressing indentations into the tubing wall of the tube to be placed into the fixing device can be easily observed and visually controlled. Furthermore, an advancement of the tube can also be controlled in a particularly easy way. The inspection window can have a simple open construction or can comprise a transparent cover that separates the contact zone from its environment and thus reduces the risk of any injuries by an unintended placement of body parts within the contact zone.

All details and embodiments of the explained flexible elongated tube can be combined in any desired way and can be transferred to the explained catheter arrangement, to the method for manufacturing a flexible elongated tube and to the indentation tool for manufacturing the flexible elongated tube in an analogous manner, and vice versa. Likewise, all details and embodiments of the catheter arrangement, the manufacturing method and the indentation tool for manufacturing a flexible and elongated tube can be combined in any desired way and can be transferred to any other of the catheter arrangement, the manufacturing method and the indentation tool in any desired combination.

Further details of aspects of the present invention will be explained with respect to exemplary embodiments and accompanying Figures. In the Figures:
- Fig. 1: shows a cross-sectional view through an embodiment of a catheter shaft;
- Fig. 2: shows a perspective view onto the catheter shaft of Figure 1;
- Fig. 3A: shows a schematic sketch of an embodiment of a catheter shaft comprising a plurality of cuts;
- Fig. 3B: shows two details of the catheter shaft of Figure 3A;
- Fig. 4A: shows an embodiment of an indentation tool;
- Fig. 4B: shows a detail of the indentation tool of Figure 4A;
- Fig. 4C: shows the indentation tool of Figure 4A in a specific operational state;
- Fig. 5A: shows a cross-sectional view through a tube which is placed in the indentation tool of Figure 4A in a first operational state of the indentation tool;
- Fig. 5B: shows a cross-sectional view through a tube which is placed in the indentation tool of Figure 4A in a second operational state of the indentation tool; and
- Fig. 5C: shows a further detail of the indentation tool of Figure 4A with an inserted tube into which indentations have been formed.

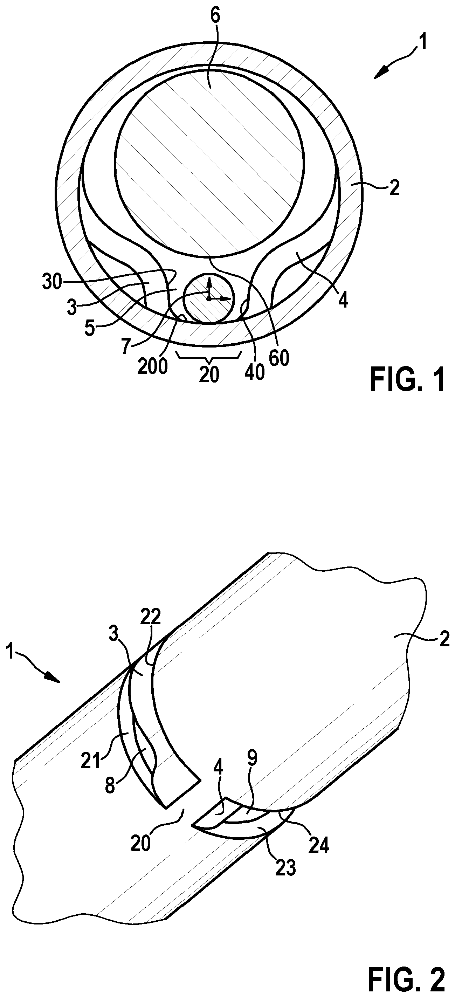

Figure 1 shows a cross-sectional view through a catheter shaft 1 that serves as flexible elongated tube. This catheter shaft 1 comprises a shaft wall 2 serving as tubing wall. Furthermore, it comprises a first push tab 3 and a second push tab 4 serving as first indentation and second indentation, respectively. The first push tab 3 and the second push tab 4 are parts of the shaft wall 2 that are pressed into an interior space of the catheter shaft 1. There is a non-impressed section 20 of the shaft wall 2 present between the first push tab 3 and the second push tab 4.

An inner surface 30 of the first push tab 3, an inner surface 40 of the second push tab 4 and an inner surface 200 of the non-impressed section 20 of the shaft wall 2 define a pull wire space 5.

Furthermore, an inner shaft 6 is arranged inside the catheter shaft 1. An outer surface 60 of this inner shaft 6 further defines a limitation of the pull wire space 5. A pull wire 7 is arranged inside the pull wire space 5. This pull wire 7 is safely guided within the pull wire space 5 by the first push tab 3 (or its inner surface 30, respectively), the second push tab 4 (or its inner surface 40, respectively), the non-impressed section 20 of the shaft wall (or its inner surface 200, respectively) and the inner shaft 6 (or its outer surface 60, respectively).

The pull wire 7 can be easily moved along a longitudinal direction (i.e., into and out of the paper plane of Figure 1) to achieve a deflection of the catheter shaft 1.

Figure 2 shows a perspective view of the catheter shaft 1 of Figure 1. The same elements are marked with the same numeral references as in Figure 1.

The first push tab 3 is formed between a first lower cut 21 of the shaft wall 2 and a first upper cut 22 of the shaft wall 2. Likewise, the second push tab 4 is formed between a second lower cut 23 and a second upper cut 24 of the shaft wall 2. There is no cut between the first push tab 3 and the non-impressed section 20 of the shaft wall 2. Likewise, there is no cut between the second push tab 4 and the non-impressed section 20 of the shaft wall 2.

Due to an indentation of the first push tab 3 and the second push tab 4 into an interior of the catheter shaft 1, a first gap 8 and a second gap 9 are formed between the push tabs 3, 4 and the shaft wall 2. A fluid communication between the interior of the catheter shaft 1 and an exterior thereof is possible through the first gap 8 and the second gap 9.

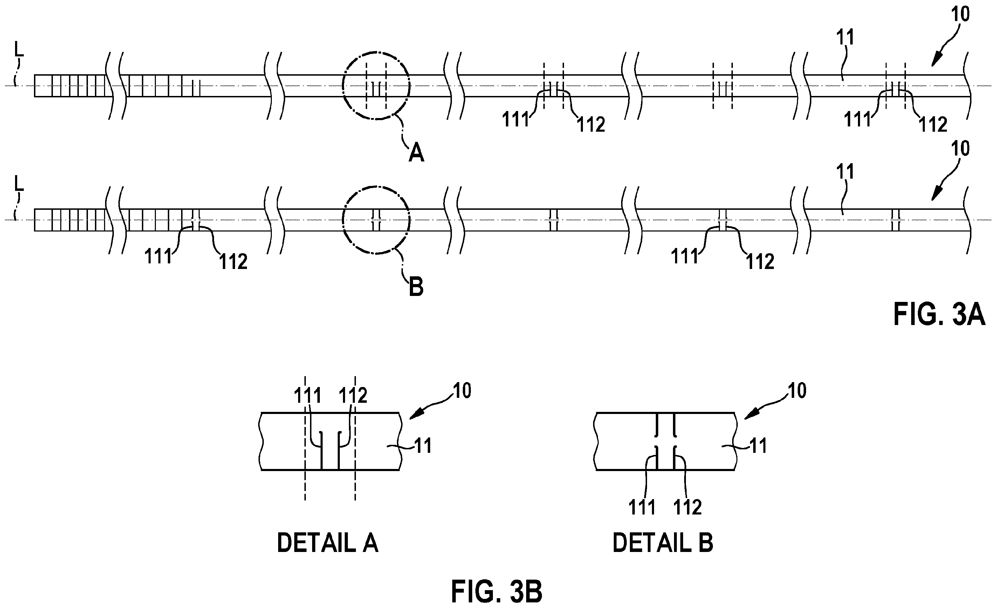

Figure 3A shows another embodiment of a catheter shaft 10 from a first side (upper panel) and from an opposite second side (lower panel). The catheter shaft 10 serves once again as flexible elongated tube and comprises a shaft wall 11 that serves as tubing wall.

There are a plurality of cuts 111 and 112 provided in regular distances along a longitudinal axis L of the catheter shaft 10. Only some of these cuts are marked with the respective numeral references. In the embodiment shown in Figure 3A, a distance between two pairs of cuts 111, 112 is approximately 100 mm. Other distances are also possible.

The cuts 111, 112 do not extend over the whole circumference of the catheter shaft 10 to avoid an undesired fragmentation of the catheter shaft 10 into a plurality of individual pieces. Thus, there always needs to be a small non-cut gap left in the areas of each pair of cuts 111, 112.

Figure 3B shows detailed views of the areas marked in Figure 3A with "A" (upper panel of Figure 3B) and "B" (lower panel of Figure 3B). These details illustrate the nature of the first cut 111 and the second cut 112 in the shaft wall 11 of the catheter shaft 10. The section of the catheter wall 11 that is arranged between the first cut 111 and the second cut 112 can be easily pushed into an interior of the catheter shaft 10 so as to form a push tab. The catheter shaft 10 of this embodiment is a so-called hypotube made from stainless steel. The first cut 111 and the second cut 112 are made by laser cutting into the metallic material of the shaft wall 11.

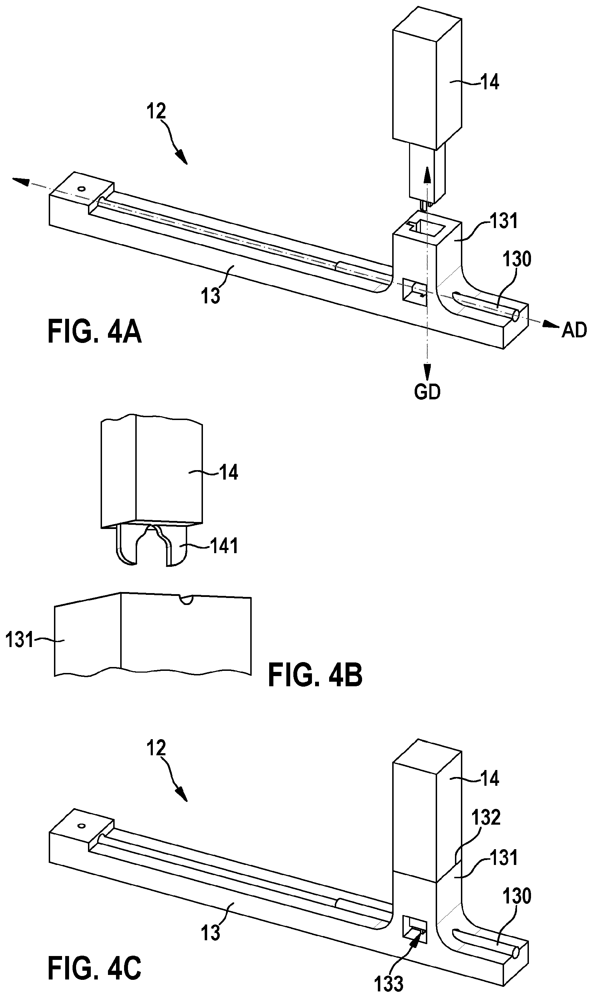

Figure 4A shows an embodiment of an indentation tool 12 that is intended to be used for making indentations into an elongated flexible tube such as a catheter shaft. This indentation tool 12 comprises a fixing device 13 that serves for aligning and fixing an elongated flexible tube. For this purpose, the fixing device 13 comprises a longitudinally extending rim 130 into which an elongated flexible tube can be inserted. Thereby, this rim 130 extends along an aligning direction AD.

The fixing device 13 further comprises a pusher guidance 131 that extends along a guidance direction GD. Thereby, the guidance direction GD extends approximately perpendicular to the aligning direction AD.

The pusher guidance 131 serves for housing a pusher 14 that can be inserted into the pusher guidance 131 and advanced in the pusher guidance 131 along the guidance direction GD.

Figure 4B shows a detail of the pusher 14 of Figure 4A. The pusher 14 comprises at its lower face side a gripper 141 with two gripping elements. This gripper 141 is intended to press push tabs into a catheter shaft or another flexible elongated tube.

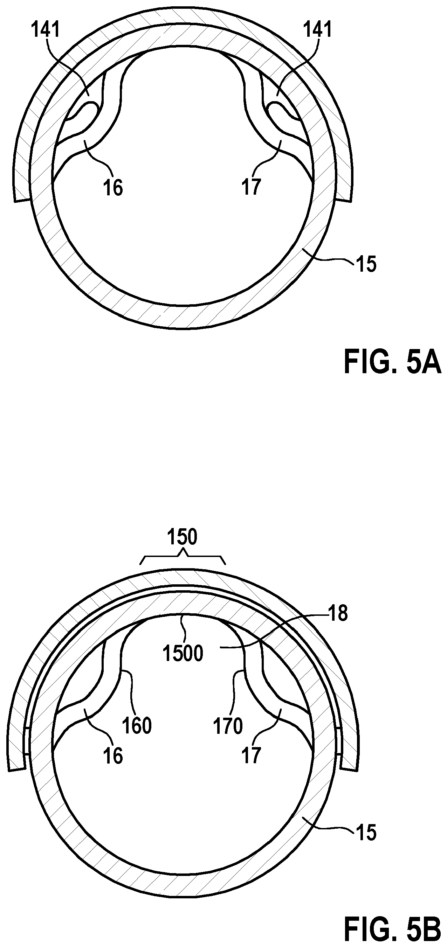

Figure 4C shows the indentation tool 12 in another operational state in which the pusher 14 is advanced as far as possible into the pusher guidance 131. The maximum movement of the pusher 14 is limited by an upper surface 132 of the pusher guidance 131 that acts as a stop of the pusher guidance 131. The gripper 141 (cf. Figure 4B) of the pusher 14 can then contact in the area of a contact zone 133 a tube being placed in the rim 130. This will be explained in more detail with respect to Figure 5A.

Figure 5A shows a cross-sectional view through a catheter shaft 15 serving as elongated flexible tube. Thereby, this catheter shaft 15 is inserted into the indentation tool 12 shown in Figures 4A to 4C. The gripper 141 is still in contact with an outer wall of the catheter shaft 15 and has pressed a first push tab 16 and a second push tab 17 into an interior of the catheter shaft 15.

Figure 5B shows the catheter shaft 15 of Figure 5A, wherein the gripper 141 has been removed from the catheter shaft 15. Nonetheless, the first push tab 16 and the second push tab 17 remain stably pressed into the interior of the catheter shaft 15. A non-impressed section 150 of the wall of the catheter shaft 15 is present between the first push tab 16 and the second push tab 17 so that a pull wire space 18 is formed between an inner surface 160 of the first push tab 16, an inner surface 170 of the second push tab 17 and an inner surface 1500 of the non-impressed section 150 of the wall of the catheter shaft 15. This pull wire space 18 serves for receiving and safely guiding a pull wire that is not yet placed into the inside of the catheter shaft 15.

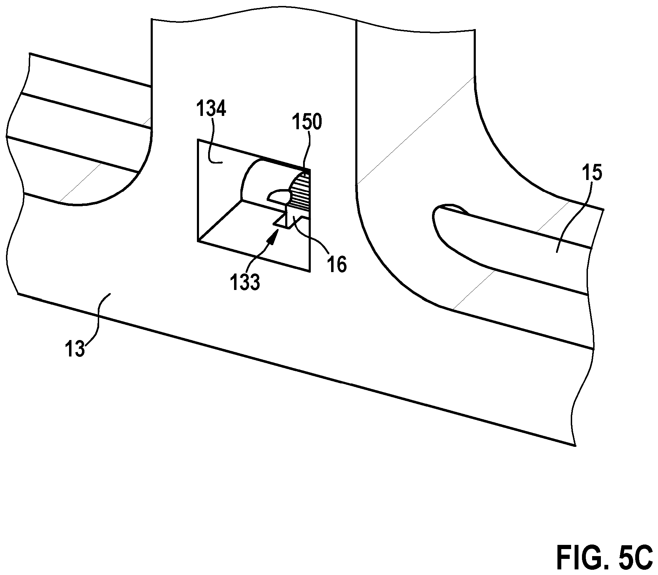

Figure 5C shows a further detail of the indentation tool 12 of Figures 4A to 4C. Thereby, an inspection window 134 is provided in the fixing device 13 so as to allow a visual inspection of the contact zone 133 (cf. also Figure 4C). Through this inspection window 134, it is possible to visually control the formation of the first push tab 16 into the catheter shaft 15. Furthermore, the non-impressed section 150 of the wall of the catheter shaft 15 can also be seen. The second push tab 17, which has also been already formed, is not visible in the depiction of Figure 5C since it is directed to the backside of the indentation tool 12.

The indentation tool 12 allows a particularly simple manufacturing of an axially aligned pair of a first push tab 16 and a second push tab 17 at the same time. By iteratively advancing the catheter shaft 15 in the fixing device 13, it is possible to produce a plurality of first push tabs and second push tabs into the wall of the catheter shaft 15. Then, a defined pull wire space or pull wire guidance is formed inside the catheter shaft 15, allowing a safe friction-less and wiggling-less guidance of a pull wire in an interior of the catheter shaft 15.

The scope of protection of the current invention is solely defined by the appended claims.

## Claims

1. Flexible elongated tube for a catheter system, comprising
a tubing wall (2);
a plurality of first indentations (3, 16) of the tubing wall (2) spaced apart from each other along a longitudinal axis (L) of the tube (1, 15); and
a plurality of second indentations (4, 17) of the tubing wall (2) spaced apart from each other along the longitudinal axis (L) of the tube (1, 15);
wherein in each case one first indentation (3, 16) and one second indentation (4, 17) form an axially aligned pair of indentations with respect to the longitudinal axis (L) of the tube (1, 15), wherein a non-impressed section (20, 150) of the tubing wall (2) is present between the first indentation (3, 16) and the second indentation (4, 17) of the axially aligned pair of indentations,
**characterized in that**
a first inner surface (30, 160) of the first indentation (3, 16) and a second inner surface (40, 170) of the second indentation (4, 17) of the axially aligned pair of indentations as well as an inner surface (200, 1500) of the non-impressed section (20, 150) of the tubing wall (2) between the first indentation (3, 16) and the second indentation (4, 17) of the axially aligned pair of indentations form a pull wire space (5, 18) that is arranged and dimensioned to receive and guide a pull wire (7) within the flexible elongated tube (1, 15).

2. Flexible elongated tube according to claim 1, **characterized in that** at least some first indentations (3, 16) of the plurality of first indentations (3, 16) and/or at least some second indentations (4, 17) of the plurality of second indentations (4, 17) are structurally identical.

3. Flexible elongated tube according to claim 1 or 2, **characterized in that** at least some first indentations (3, 16) of the plurality of first indentations (3, 16) and/or at least some second indentations (4, 17) of the plurality of second indentations (4, 17) are separated from axially adjacent non-impressed sections of the flexible elongated tube (1, 15) by a cut (21, 22, 23, 24).

4. Flexible elongated tube according to claim 3, **characterized in that** a gap (8, 9) is formed between at least some first indentations (3, 16) of the plurality of first indentations (3, 16) and axially adjacent non-impressed sections of the flexible elongated tube (1, 15) and/or between at least some second indentations (4, 17) of the plurality of second indentations (4, 17) and axially adjacent non-impressed sections of the flexible elongated tube (1, 15), wherein the gap (8, 9) provides a fluid communication between an interior of the flexible elongated tube (1, 15) and an exterior thereof.

5. Catheter arrangement comprising a flexible elongated tube (1, 15) according to any of the preceding claims and a pull wire (7),
**characterized in that**
the pull wire (7) is received and guided within the flexible elongated tube (1, 15) in the pull wire space (5, 18) formed by the first inner surface (30, 160) of the first indentation (3, 16) and the second inner surface (40, 170) of the second indentation (4, 17) of the axially aligned pair of indentations as well as by the inner surface (200, 1500) of the non-impressed section (20, 150) of the tubing wall (2) between the first indentation (3, 16) and the second indentation (4, 17) of the axially aligned pair of indentations.

6. Catheter arrangement according to claim 5, **characterized in that** an inner tube (6) is additionally received in an interior of the flexible elongated tube (1).

7. Catheter arrangement according to claim 6, **characterized in that** an outer surface (60) of the inner tube (6) forms an additional wall of the pull wire space (5).

8. Method for manufacturing a flexible elongated tube according to any of claims 1 to 4, comprising the following steps:
a) placing a flexible elongated tube into a fixing device (13) so as to fix and align the flexible elongated tube at least section-wise in an oblong aligning direction (AD);
b) placing a pusher (14) into a pusher guidance (131) of the fixing device (13);
c) advancing the pusher (14) towards the flexible elongated tube in a guidance direction (GD) within the pusher guidance (131), wherein the guidance direction (GD) is essentially perpendicular to the aligning direction (AD);
d) contacting the flexible elongated tube with a gripper (141) arranged on an end face of the pusher (14);
e) further advancing the pusher (14) until it contacts a stop (132) of the pusher guidance (131), thereby automatically exerting a pressure onto the flexible elongated tube leading to formation of a first indentation (3, 16) and a second indentation (4, 17) that are axially aligned with respect to a longitudinal axis (L) of the flexible elongated tube (1, 15);
f) retracting the pusher (14) away from the flexible elongated tube (1, 15);
g) advancing the flexible elongated tube (1, 15) within the fixing device (13);
h) repeatedly carrying out steps c) to g) until a desired number of first indentations (3, 16) and second indentations (4, 17) is formed in the flexible elongated tube (1, 15).

9. Method according to claim 8, **characterized in that** the flexible elongated tube (1, 15) is advanced within the fixing device (13) by a pre-definable amount.

10. Method according to claim 9, **characterized in that** the pre-definable amount remains constant for a plurality of subsequent advancing steps.

11. Method according to any of claims 8 to 10, **characterized in that** the pusher (14) is at least partially advanced with a clamp.

12. Method according to any of claims 8 to 11, **characterized in that** the first indentation (3, 16) and the second indentation (4, 17) are formed in an area of the flexible elongated tube (1, 15) between at least two axially spaced cuts (21, 22; 23, 24) around a section of a circumference of the flexible elongated tube (1, 15).

13. Indentation tool for carrying out the method according to any of claims 8 to 12, comprising
a fixing device (13) for fixing and aligning a flexible elongated tube at least section-wise in an oblong aligning direction (AD), the fixing device (13) further comprising a pusher guidance (131) for guiding a pusher (14) in a guidance direction (GD), wherein the guidance direction (GD) is essentially perpendicular to the aligning direction (AD);
a pusher (14) that can be moved in the pusher guidance (131) along the guidance direction (GD), wherein the pusher (14) comprises a gripper (141) arranged on an end face of the pusher (14), the gripper (141) being arranged and designed for making indentations (3, 4, 16, 17) into a flexible elongated tube arranged in the fixing device (13);
wherein the pusher guidance (131) comprises a stop (132) for limiting the maximum possible movement of the pusher (14) in the pusher guidance (131) in the guidance direction (GD) towards the fixing device (13).

14. Indentation tool according to claim 13, **characterized in that** the fixing device (13) comprises an inspection window (134) allowing for a visual control of a contact zone (133) in which the gripper (141) can contact a flexible elongated tube arranged in the fixing device (13) during intended operation of the indentation tool (12).

## Patentansprüche

1. Flexibler, länglicher Schlauch für ein Kathetersystem, umfassend
eine Schlauchwand (2);
eine Vielzahl von ersten Vertiefungen (3, 16) der Schlauchwand (2), die entlang einer Längsachse (L) des Schlauchs (1, 15) voneinander beabstandet sind; und
eine Vielzahl von zweiten Vertiefungen (4, 17) der Schlauchwand (2), die entlang der Längsachse (L) des Schlauchs (1, 15) voneinander beabstandet sind;
wobei jeweils eine erste Vertiefung (3, 16) und eine zweite Vertiefung (4, 17) ein axial ausgerichtetes Paar von Vertiefungen in Bezug auf die Längsachse (L) des Schlauchs (1, 15) bilden, wobei ein nicht eingeprägter Abschnitt (20, 150) der Schlauchwand (2) zwischen der ersten Vertiefung (3, 16) und der zweiten Vertiefung (4, 17) des axial ausgerichteten Paares von Vertiefungen vorhanden ist,
**dadurch gekennzeichnet, dass**
eine erste Innenfläche (30, 160) der ersten Vertiefung (3, 16) und eine zweite Innenfläche (40, 170) der zweiten Vertiefung (4, 17) des axial ausgerichteten Paares von Vertiefungen sowie eine Innenfläche (200, 1500) des nicht eingeprägten Abschnitts (20, 150) der Schlauchwand (2) zwischen der ersten Vertiefung (3, 16) und der zweiten Vertiefung (4, 17) des axial ausgerichteten Paares von Vertiefungen einen Zugdrahtraum (5, 18) bilden, der so angeordnet und dimensioniert ist, dass er einen Zugdraht (7) innerhalb des flexiblen länglichen Schlauchs (1, 15) aufnehmen und führen kann .

2. Flexibler länglicher Schlauch nach Anspruch 1, **dadurch gekennzeichnet, dass** zumindest einige erste Vertiefungen (3, 16) der Vielzahl von ersten Vertiefungen (3, 16) und/oder zumindest einige zweite Vertiefungen (4, 17) der Vielzahl von zweiten Vertiefungen (4, 17) strukturell identisch sind.

3. Flexibler länglicher Schlauch gemäß Anspruch 1 oder 2, **dadurch gekennzeichnet, dass** zumindest einige erste Vertiefungen (3, 16) der Vielzahl von ersten Vertiefungen (3, 16) und/oder zumindest einige zweite Vertiefungen (4, 17) der Vielzahl von zweiten Vertiefungen (4, 17) durch einen Schnitt (21, 22, 23, 24) von axial benachbarten, nicht eingeprägten Abschnitten des flexiblen länglichen Schlauchs (1, 15) getrennt sind.

4. Flexibler, länglicher Schlauch gemäß Anspruch 3, **dadurch gekennzeichnet, dass** zwischen zumindest einigen ersten Vertiefungen (3, 16) der Vielzahl von ersten Vertiefungen (3, 16) und axial benachbarten, nicht eingeprägten Abschnitten des flexiblen, länglichen Schlauchs (1, 15) und/oder zwischen zumindest einigen zweiten Vertiefungen (4, 17) der Vielzahl von zweiten Vertiefungen (4, 17) und axial benachbarten, nicht eingeprägten Abschnitten des flexiblen länglichen Schlauchs (1, 15) ein Spalt (8, 9) gebildet ist, wobei der Spalt (8, 9) eine Fluidverbindung zwischen einem Inneren des flexiblen länglichen Schlauchs (1, 15) und einem Äußeren davon bereitstellt.

5. Katheteranordnung, umfassend einen flexiblen länglichen Schlauch (1, 15) gemäß einem der vorstehenden Ansprüche und einen Zugdraht (7),
**dadurch gekennzeichnet, dass**
der Zugdraht (7) innerhalb des flexiblen länglichen Schlauchs (1, 15) in dem Zugdrahtraum (5, 18) aufgenommen und geführt wird, der durch die erste Innenfläche (30, 160) der ersten Vertiefung (3, 16) und die zweite Innenfläche (40, 170) der zweiten Vertiefung (4, 17) des axial ausgerichteten Paares von Vertiefungen sowie durch die Innenfläche (200, 1500) des nicht eingeprägten Abschnitts (20, 150) der Schlauchwand (2) zwischen der ersten Vertiefung (3, 16) und der zweiten Vertiefung (4, 17) des axial ausgerichteten Paares von Vertiefungen gebildet wird.

6. Katheteranordnung nach Anspruch 5, **dadurch gekennzeichnet, dass** zusätzlich ein Innenschlauch (6) in einem Innenraum des flexiblen länglichen Schlauchs (1) aufgenommen ist.

7. Katheteranordnung nach Anspruch 6, **dadurch gekennzeichnet, dass** eine Außenfläche (60) des Innenschlauchs (6) eine zusätzliche Wand des Zugdrahtraums (5) bildet.

8. Verfahren zur Herstellung eines flexiblen länglichen Schlauchs gemäß einem der Ansprüche 1 bis 4 mit den folgenden Schritten:
a) Anordnen eines flexiblen länglichen Schlauchs in einer Befestigungsvorrichtung (13), um den flexiblen länglichen Schlauch zumindest abschnittsweise in einer Längsausrichtung (AD) zu befestigen und auszurichten;
b) Anordnen eines Schiebers (14) in einer Schieberführung (131) der Befestigungsvorrichtung (13);
c) Vorschieben des Schiebers (14) in einer Führungsrichtung (GD) innerhalb der Schieberführung (131) in Richtung des flexiblen länglichen Schlauchs, wobei die Führungsrichtung (GD) im Wesentlichen senkrecht zur Längsausrichtung (AD) ist;
d) Inkontaktbringen des flexiblen, länglichen Schlauchs mit einer Greifvorrichtung (141), die an einer Stirnseite des Schiebers (14) angeordnet ist;
e) weiteres Vorschieben des Schiebers (14), bis er einen Anschlag (132) der Schieberführung (131) berührt, wodurch automatisch ein Druck auf den flexiblen länglichen Schlauch ausgeübt wird, was zur Bildung einer ersten Vertiefung (3, 16) und einer zweiten Vertiefung (4, 17) führt, die in Bezug auf eine Längsachse (L) des flexiblen länglichen Schlauchs (1, 15) axial ausgerichtet sind;
f) Zurückziehen des Schiebers (14) vom flexiblen länglichen Schlauch (1, 15);
g) Vorschieben des flexiblen länglichen Schlauchs (1, 15) innerhalb der Befestigungsvorrichtung (13);
h) wiederholtes Ausführen der Schritte c) bis g), bis eine gewünschte Anzahl von ersten Vertiefungen (3, 16) und zweiten Vertiefungen (4, 17) in dem flexiblen länglichen Schlauch (1, 15) gebildet ist.

9. Verfahren nach Anspruch 8, **dadurch gekennzeichnet, dass** der flexible längliche Schlauch (1, 15) um einen vordefinierbaren Weg innerhalb der Befestigungsvorrichtung (13) vorgeschoben wird.

10. Verfahren nach Anspruch 9, **dadurch gekennzeichnet, dass** der vordefinierbare Weg für eine Vielzahl aufeinanderfolgender Vorschubschritte konstant bleibt.

11. Verfahren nach einem der Ansprüche 8 bis 10, **dadurch gekennzeichnet, dass** der Schieber (14) zumindest teilweise mit einer Klemme vorgeschoben wird.

12. Verfahren nach einem der Ansprüche 8 bis 11, **dadurch gekennzeichnet, dass** die erste Vertiefung (3, 16) und die zweite Vertiefung (4, 17) in einem Bereich des flexiblen länglichen Schlauchs (1, 15) zwischen mindestens zwei axial beabstandeten Schnitten (21, 22; 23, 24) um einen Abschnitt eines Umfangs des flexiblen länglichen Schlauchs (1, 15) herum ausgebildet sind.

13. Einpresswerkzeug zur Durchführung des Verfahrens gemäß einem der Ansprüche 8 bis 12, umfassend
eine Befestigungsvorrichtung (13) zum Befestigen und Ausrichten eines flexiblen länglichen Schlauchs zumindest abschnittsweise in einer Längsausrichtung (AD), wobei die Befestigungsvorrichtung (13) ferner eine Schieberführung (131) zum Führen eines Schiebers (14) in einer Führungsrichtung (GD) umfasst, wobei die Führungsrichtung (GD) im Wesentlichen senkrecht zur Längsausrichtung (AD) ist;
einen Schieber (14), der in der Schieberführung (131) entlang der Führungsrichtung (GD) bewegbar ist, wobei der Schieber (14) eine an einer Stirnseite des Schiebers (14) angeordnete Greifvorrichtung (141) umfasst, wobei die Greifvorrichtung (141) so angeordnet und ausgebildet ist, dass sie Vertiefungen (3, 4, 16, 17) in einem in der Befestigungsvorrichtung (13) angeordneten flexiblen länglichen Schlauch erzeugt;
wobei die Schieberführung (131) einen Anschlag (132) umfasst, um die maximal mögliche Bewegung des Schiebers (14) in der Schieberführung (131) in Führungsrichtung (GD) in Richtung der Befestigungsvorrichtung (13) zu begrenzen.

14. Einpresswerkzeug nach Anspruch 13, **dadurch gekennzeichnet, dass** die Befestigungsvorrichtung (13) ein Sichtfenster (134) umfasst, das eine visuelle Kontrolle einer Kontaktzone (133) ermöglicht, in der die Greifvorrichtung (141) während des vorgesehenen Betriebs des Einpresswerkzeugs mit einem in der Befestigungsvorrichtung (13) angeordneten flexiblen länglichen Schlauch in Kontakt kommen kann. (12)

## Revendications

1. Tube flexible allongé pour système de cathéter, comprenant
une paroi de tube (2) ;
une pluralité de premières indentations (3, 16) de la paroi de tube (2) espacées les unes des autres le long d'un axe longitudinal (L) du tube (1, 15) ; et
une pluralité de secondes indentations (4, 17) de la paroi de tube (2) espacées les unes des autres le long de l'axe longitudinal (L) du tube (1, 15) ;
dans lequel, dans chaque cas, une première indentation (3, 16) et une seconde indentation (4, 17) forment une paire d'indentations alignées axialement par rapport à l'axe longitudinal (L) du tube (1, 15), dans lequel une section non empreinte (20, 150) de la paroi de tube (2) est présente entre la première indentation (3, 16) et la seconde indentation (4, 17) de la paire d'indentations alignées axialement,
**caractérisé en ce que**
une première surface intérieure (30, 160) de la première indentation (3, 16) et une seconde surface intérieure (40, 170) de la seconde indentation (4, 17) de la paire d'indentations alignées axialement ainsi qu'une surface intérieure (200, 1500) de la section non empreinte (20, 150) de la paroi de tube (2) entre la première indentation (3, 16) et la seconde indentation (4, 17) de la paire d'indentations alignées axialement forment un espace pour fil de traction (5, 18) qui est agencé et dimensionné pour recevoir et guider un fil de traction (7) à l'intérieur du tube flexible allongé (1, 15).

2. Tube flexible allongé selon la revendication 1, **caractérisé en ce qu'**au moins certaines premières indentations (3, 16) de la pluralité de premières indentations (3, 16) et/ou au moins certaines secondes indentations (4, 17) de la pluralité de secondes indentations (4, 17) sont structurellement identiques.

3. Tube flexible allongé selon la revendication 1 ou 2, **caractérisé en ce qu'**au moins certaines premières indentations (3, 16) de la pluralité de premières indentations (3, 16) et/ou au moins certaines secondes indentations (4, 17) de la pluralité de secondes indentations (4, 17) sont séparées de sections non empreinte axialement adjacentes du tube flexible allongé (1, 15) par une découpe (21, 22, 23, 24).

4. Tube flexible allongé selon la revendication 3, **caractérisé en ce qu'**un espace (8, 9) est formé entre au moins certaines premières indentations (3, 16) de la pluralité de premières indentations (3, 16) et des sections non empreintes axialement adjacentes du tube flexible allongé (1, 15) et/ou entre au moins certaines secondes indentations (4, 17) de la pluralité de secondes indentations (4, 17) et des sections non empreintes axialement adjacentes du tube flexible allongé (1, 15), l'espace (8, 9) fournissant une communication de fluide entre un intérieur du tube flexible allongé (1, 15) et un extérieur de celui-ci.

5. Agencement de cathéter comprenant un tube flexible allongé (1, 15) selon l'une quelconque des revendications précédentes et un fil de traction (7),
**caractérisé en ce que**
le fil de traction (7) est reçu et guidé à l'intérieur du tube flexible allongé (1, 15) au sein de l'espace de fil de traction (5, 18) formé par la première surface intérieure (30, 160) de la première indentation (3, 16) et la seconde surface intérieure (40, 170) de la seconde indentation (4, 17) de la paire d'indentations alignées axialement ainsi que par la surface intérieure (200, 1500) de la section non empreinte (20, 150) de la paroi de tube (2) entre la première indentation (3, 16) et la seconde indentation (4, 17) de la paire d'indentations alignées axialement.

6. Agencement de cathéter selon la revendication 5, **caractérisé en ce qu'**un tube interne (6) est en outre reçu à l'intérieur du tube flexible allongé (1).

7. Agencement de cathéter selon la revendication 6, **caractérisé en ce qu'**une surface extérieure (60) du tube intérieur (6) forme une paroi supplémentaire de l'espace de fil de traction (5).

8. Procédé de fabrication d'un tube flexible allongé selon l'une quelconque des revendications 1 à 4, comprenant les étapes suivantes consistant à :
a) placer un tube flexible allongé dans un dispositif de fixation (13) de manière à fixer et aligner le tube flexible allongé, au moins par sections, dans une direction d'alignement oblongue (AD) ;
b) placer un poussoir (14) dans un élément de guidage de poussoir (131) du dispositif de fixation (13) ;
c) faire avancer le poussoir (14) vers le tube flexible allongé dans une direction de guidage (GD) à l'intérieur de l'élément de guidage de poussoir (131), la direction de guidage (GD) étant essentiellement perpendiculaire à la direction d'alignement (AD) ;
d) mettre en contact le tube flexible allongé avec une pince (141) disposée sur une face d'extrémité du poussoir (14) ;
e) faire avancer davantage le poussoir (14) jusqu'à ce qu'il entre en contact avec une butée (132) de l'élément de guidage de poussoir (131), en exerçant ainsi automatiquement une pression sur le tube flexible allongé qui conduit à la formation d'une première indentation (3, 16) et d'une seconde indentation (4, 17) qui sont alignées axialement par rapport à un axe longitudinal (L) du tube flexible allongé (1, 15) ;
f) rétracter le poussoir (14) en l'éloignant du tube flexible allongé (1, 15) ;
g) faire avancer le tube flexible allongé (1, 15) à l'intérieur du dispositif de fixation (13) ;
h) effectuer de manière répétée les étapes c) à g) jusqu'à ce qu'un nombre souhaité de premières indentations (3, 16) et de secondes indentations (4, 17) soit formé dans le tube flexible allongé (1, 15).

9. Procédé selon la revendication 8, **caractérisé en ce que** le tube flexible allongé (1, 15) est avancé à l'intérieur du dispositif de fixation (13) d'une quantité prédéfinissable.

10. Procédé selon la revendication 9, **caractérisé en ce que** la quantité prédéfinissable reste constante pour une pluralité d'étapes d'avancement ultérieures.

11. Procédé selon l'une quelconque des revendications 8 à 10, **caractérisé en ce que** le poussoir (14) est au moins partiellement avancé avec une pince de serrage.

12. Procédé selon l'une quelconque des revendications 8 à 11, **caractérisé en ce que** la première indentation (3, 16) et la seconde indentation (4, 17) sont formées dans une zone du tube flexible allongé (1, 15) entre au moins deux découpes espacées axialement (21, 22 ; 23, 24) autour d'une section d'une circonférence du tube flexible allongé (1, 15).

13. Outil d'indentation pour la mise en œuvre du procédé selon l'une quelconque des revendications 8 à 12, comprenant
un dispositif de fixation (13) permettant de fixer et d'aligner un tube flexible allongé au moins par sections dans une direction d'alignement oblongue (AD), le dispositif de fixation (13) comprenant en outre un élément de guidage de poussoir (131) permettant de guider un poussoir (14) dans une direction de guidage (GD), la direction de guidage (GD) étant essentiellement perpendiculaire à la direction d'alignement (AD) ;
un poussoir (14) qui peut être déplacé dans l'élément de guidage de poussoir (131) le long de la direction de guidage (GD), le poussoir (14) comprenant une pince (141) disposée sur une face d'extrémité du poussoir (14), la pince (141) étant agencée et conçue pour réaliser des indentations (3, 4, 16, 17) dans un tube flexible allongé agencé dans le dispositif de fixation (13) ;
dans lequel l'élément de guidage de poussoir (131) comprend une butée (132) permettant de limiter le déplacement maximum possible du poussoir (14) dans l'élément de guidage de poussoir (131) dans la direction de guidage (GD) vers le dispositif de fixation (13).

14. Outil d'indentation selon la revendication 13, **caractérisé en ce que** le dispositif de fixation (13) comprend une fenêtre d'inspection (134) permettant un contrôle visuel d'une zone de contact (133) dans laquelle la pince (141) peut venir en contact avec un tube flexible allongé agencé dans le dispositif de fixation (13) pendant le fonctionnement prévu de l'outil d'indentation (12).
